# EUROPÄISCHE PATENTSCHRIFT

(11) **EP 0 411 417 B1**
(45) Veröffentlichungstag und Bekanntmachung des Hinweises auf die Patenterteilung: **10.11.1993**
(21) Anmeldenummer: 90114012.9
(22) Anmeldetag: 21.07.1990
(51) Int. Cl.: C07C 229/30, C07C 255/30, C07C 227/16, C07C 253/30

(54) **Verfahren zur Herstellung von Aminomethylenverbindungen**
Process for preparing aminomethylene compounds
Procédé pour la préparation de composés aminométhyléniques

(30) Priorität: 03.08.1989 DE 3925720
(43) Veröffentlichungstag der Anmeldung: 06.02.1991
(73) Patentinhaber: BAYER AG, 51368 Leverkusen (DE)
(72) Erfinder: Blank, Heinz-Ulrich, Dr., D-5068 Odenthal (DE); Kraus, Helmut, Dr., D-5000 Köln 80 (DE)

(56) Entgegenhaltungen:
- DD-A- 151 624
- DD-A- 257 067
- GB-A- 917 436
- CHEMISCHE BERICHTE, Band 96, 1963, Weinheim H. BREDEREK et al. " ber SUureamid-Dialkylsulfat- Komplexe" Seiten 1350-1355
- CHEMICAL ABSTRACTS, Band 93, Nr. 5, 4. August 1980 Columbus, Ohio, USA W. KANTLEHNER et al. "Orthoamides. XXXII. Reaction of tert-butoxy-N,N, N',N'-tetramethylmethane- diamine with NH- and CH- -acidic compounds" Seite 855, Spalte 2, Zusammenfassung-Nr. 45 870v

## Beschreibung

Die Erfindung betrifft die Herstellung von Aminomethylenverbindungen durch Aminomethylenierung von C-H-aciden Verbindungen in einer einstufigen Reaktion. Aminomethylenverbindungen, wie aminomethylenierte Dinitrile, Cyanessigester und Malonester, sind wichtige C₃- bzw. C₄-Bausteine bei der Synthese von Heterocyclen, wie beispielsweise Pyrazol-, Pyridin- und Chinolinderivaten, die als pharmazeutische Wirkstoffe und Pflanzenschutzmittel Verwendung finden (US 4.620.865).

Es ist bekannt, daß man C-H-acide Verbindungen durch Umsetzung mit Dimethylformamid (DMF) und Hilfsstoffen aminomethylenieren kann. Verwendet man beispielsweise einen zehnfachen molaren Überschuß an Acetanhydrid als Hilfsstoff, so erhält man nach 5- bis 7-stündigem Erhitzen unter Rückfluß Ausbeuten von 20 bis 40 % der theoretischen Ausbeute an aminomethylenierten Substanzen (Arch. Pharm. 295 (1962), 516). Bei Variation der Reaktionsbedingungen zur Verbesserung der Raum-Zeit-Ausbeute entstehen gemäß DD 151 624 größere Mengen an Kohlenmonoxid. Ersetzt man das Acetanhydrid durch POCl₃ und arbeitet in überschüssigem DMF, werden beim Einsatz von Cyanessigsäuremethylester als C-H-acide Verbindung etwa 47 % Reaktionsprodukt erhalten (Z. obs. chim. 32 (1962), 4050, zitiert in DD 151 624). Die Reaktionsführung und die Aufarbeitung sind sehr aufwendig. Wird die genannte Variante mit POCl₃ in Benzol durchgeführt, so werden nur 7 % Reaktionsprodukt isoliert (Chem. Ber. 94 (1961), 2278). Verwendet man Chlorameisensäureethylester als Hilfsstoff, so werden im Falle des Cyanessigesters wegen eintretender Nebenreaktionen nur 31 % der theoretischen Ausbeute erhalten. Die Verwendung von Phosgen ergibt im Falle des Malonsäurediethylesters unter Verwendung eines Überschusses an Natriummalonester als Base 81 % der theoretischen Ausbeute. Beim Cyanessigsäureethylester werden mit Triethylamin als Base 75 % der theoretischen Ausbeute erhalten (Chem. Ber. 94 (1961), 2278). Jedoch ist die Verwendung von Natriummalonester kostspielig, mit Triethylamin hingegen müssen aufwendig große Mengen Triethylaminhydrochlorid abgetrennt werden.

Gemäß DD 257 067 führt die Reaktion von Cyanacetat mit dem DMF-Dimethylsulfat-Addukt und Natriumcarbonat als Base zu 53-61 % des aminomethylenierten Produkts. Der uneinheitliche Schmelzpunkt weist jedoch auf Verunreinigungen hin.

Ohne Base läßt sich die Aminomethylenierung mit Thionylchlorid durchführen. Dabei muß in Tetrachlorkohlenstoff 5 Stunden unter Rückflußbedingungen erhitzt werden, um eine Ausbeute von 79 % Dimethylamino-cyanessigsäureethylester zu erhalten. Allerdings muß das schwarze Rohprodukt umkristallisiert werden, so daß im günstigsten Fall (bei 25 %igem Überschuß an DMF und Thionylchlorid) 68 % der theoretischen Ausbeute an sauberer Substanz isoliert werden kann (DD 151 624). Eigene Versuche zeigten, daß bei Verwendung des weniger problematischen Lösungsmittels Toluol nochmals 15 % weniger Reaktionsprodukt erhalten werden. Eine Aminomethylenierung von Malonester ist nach dieser Methode nicht möglich.

Außerdem wird bei Umsetzungen mit DMF und Chlorierungsmitteln, wie PCl₅, POCl₃, SOCl₂, COCl₂ und ähnlichen, als Nebenkomponente das kanzerogene N,N-Dimethylcarbamidchlorid gebildet, so daß die Handhabung und Verwendung der so hergestellten Produkte problematisch ist; eine Verwendung ist erst nach aufwendigen Reinigungsschritten möglich.

Die Aminomethylenierung mit der Kombination Gold'sches Salz/Natriummethylat führt nach Erhitzen zum Rückfluß über Nacht in Ethanol zu einer Ausbeute von 55 bis 82 % der theoretischen Ausbeute (Synth. Commun. 12 (1982), 939). Die Aufarbeitung ist aufwendig, dennoch muß das Reaktionsprodukt zusätzlich umkristallisiert werden. Zur Herstellung des Gold'schen Salzes wird ein hoher Überschuß an DMF benötigt. Dies und auch die Isolierung des Salzes ist relativ kostenintensiv, so daß eine technische Realisierung zu teuer wird.

Die Aminomethylenierung mit Amidinen oder mit der Kombination Orthoester/sek.-Amin ist wegen der teuren Reagenzien unwirtschaftlich. Kostenungünstig ist auch die Kondensation mit Amidacetalen und Aminalestern wegen der umständlichen Darstellungsweise dieser Orthoamide. So werden DMF-Acetale durch Alkylierung mit Alkylsulfaten (Chem. Ber. 96 (1963), 1350) und anschließende Umsetzung mit Alkalialkoholaten hergestellt (Chem. Ber. 101 (1968), 41). Die Isolierung aus dem alkoholischen Reaktionsgemisch ist gemäß DD 94 359 umständlich und zeitraubend, Ein in diesem DD-Patent erwähntes, vermeintlich verbessertes Destillationsverfahren wird in der bereits oben erwähnten DD 151 624 als technisch schwierig realisierbar bezeichnet. Außerdem konnte bei einer labormäßigen Nacharbeitung die angegebene hohe Ausbeute nicht erreicht werden.

Die Aminalester sind zugänglich durch Umsetzung der DMF-Dialkylsulfat-Addukte mit Dimethylamin und anschließend mit Alkalimetallalkoholaten in Hexan, Cyclohexan oder Ether (Chem. Ber. 101 (1968), 41). Die Ausbeuten betragen bei Kondensationen von C-H-aciden Verbindungen mit Dimethylformamid-Diethylacetal 77 bis 84 %, mit dem Methylaminalester maximal 89 %; das Acetal ist in 63 %iger theoretischer Ausbeute aus dem DMF-Dimethyl-sulfat-Addukt zugänglich, während der Methylaminalester in 62 %iger theoretischer Ausbeute, ausgehend von Tetramethyl-formamidinium-methylsulfat erhältlich ist.

Ein weiteres Verfahren zur Herstellung von substituierten Aminomethylenverbindungen ist aus GB 917 436 bekannt. Hierbei werden CH-acide Ester und Ketone mit Amidchloriden der Formel [(A,B)N=CH-Cl]^{⊕}Z^{⊖} umgesetzt. Amidchloride, in denen Z^{⊖} Chlorid bedeutet, können aus DMF und Phosgen erhalten werden; dieser Weg ist jedoch schwierig und führt über cancerogene Carbamoylchloride.

Es wurde ein Verfahren zur Herstellung von Aminomethylenverbindungen der Formel 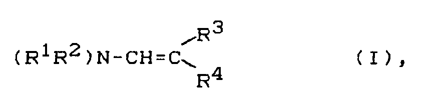
in der
- R¹ und R²: unabhängig voneinander geradkettiges oder verzweigtes C₁-C₈-Alkyl, C₂-C₈-Alkenyl, C₂-C₈-Alkoxyalkyl,C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R¹ und R² gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann, und
- R³ und R⁴: unabhängig voneinander C₆-C₁₂-Aryl, -NO₂, -CN, -NC, COR⁵, CSR⁵, CO-OR⁵, CO-SR⁵ oder CO-N(R⁵,R⁶) bedeuten, worin R⁵ und R⁶ den für R¹ und R² genannten Bedeutungsumfang annehmen, jedoch unabhängig von R¹ und R² sind und zusätzlich Wasserstoff bedeuten können,

gefunden, das dadurch gekennzeichnet ist, daß man C-H-acide Verbindungen der Formel 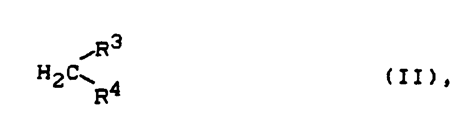
in der
- R³ und R⁴: die genannte Bedeutung haben,

mit Salzen der Formel 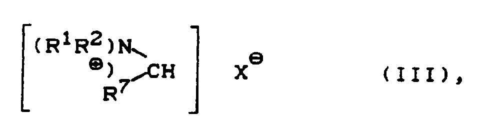
in der
- R¹ und R²: die genannte Bedeutung haben,
- R⁷: für -OR⁸ oder -N(R⁸, R⁹) steht, worin R⁸ und R⁹ unabhängig voneinander und unabhängig von R¹ und R² den für R¹ und R² genannten Bedeutungsumfang haben und
- X^{⊖}: das C₁-C₈-Alkylsulfatanion, das C₆-C₁₂-Arylsulfonatanion, das Tetrafluoroboranatanion oder das Hexachloroantimonatanion bedeutet,

in Gegenwart von Alkoholaten der Formel

M¹OR¹⁰ (IV),

in der
- R¹⁰: geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₂-C₈-Alkylen-OM¹ oder C₇-C₁₀-Aralkyl bedeutet und
- M¹: ein Äquivalent eines Alkalimetall- oder Erdalkalimetallkations ist,

in einer einstufigen Reaktion bei einer Temperatur von 10 bis 70°C, bevorzugt 20 bis 60°C, umsetzt.

Geradkettiges oder verzweigtes C₁-C₈-Alkyl ist beispielsweise Methyl, Ethyl, Propyl, Isopropyl, Butyl, Isobutyl, tert.-Butyl, die isomeren Amyle, Hexyle, Octyle, bevorzugt die genannten C₁-C₄-Alkylreste.

C₂-C₈-Alkenyl ist Vinyl, Propenyl, Allyl, die isomeren Butenyle, Amylenyle, Hexenyle oder Octenyle, bevorzugt die genannten C₃-C₄-Alkenylreste.

C₂-C₈-Alkoxyalkyl ist beispielsweise Methoxymethyl, Ethoxymethyl, Methoxyethyl sowie weitere Reste aus der Gruppe C₃-C₈-Alkyl, in welchem ein C-Atom durch ein O-Atom ersetzt ist.

C₃-C₈-Alkoxyalkenyl ist beispielsweise Methoxyvinyl, Ethoxyvinyl, Methoxyallyl, 2-Methoxy-propenyl und andere aus der Gruppe von C₄-C₈-Alkenyl, worin ein C-Atom durch ein O-Atom ersetzt ist.

C₃-C₈-Cycloalkyl ist beispielsweise Cyclopropyl, Methyl-cyclopropyl, Dimethyl-cyclopropyl, Cyclobutyl, Methyl-cyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenyl-ethyl, 2-Phenyl-ethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, Morpholin, Pyran, Azepin, Azocin, Isoxazol, Isothiazol, Pyridazin und Pyrazin.

Es ist dem Fachmann bekannt, daß ungesättigte heterocyclische Ringe einen mehr oder weniger stark ausgeprägten aromatischen Charakter haben können.

Weiterhin können R¹ und R² gemeinsam mit dem N-Atom, das sie substituieren, einen 5- bis 8-gliedrigen gesättigten oder ungesättigten (gegebenenfalls aromatischen) N-heterocyclischen Ring bilden, der ein weiteres Heteroatom aus der Gruppe N, O und S enthalten kann. Solche Systeme sind beispielsweise Pyrrol, Pyrrolidin, Pyrrolin, Pyrazol, Pyrazolidin, Imidazol, Imidazolidin, Thiazol, Thiazolidin, Piperazin, Piperidin, Morpholin, Azepin, Dihydroazocin.

Das C₁-C₈-Alkylsulfatanion ist beispielsweise das Anion von Methylschwefelsäure, Ethylschwefelsäure, Propylschwefelsäure, Isopropylschwefelsäure, Butylschwefelsäure, Isobutylschwefelsäure, eine der isomeren Hexylschwefelsäuren oder Octylschwefelsäuren.

Das C₆-C₁₂-Arylsulfonatanion ist beispielsweise das Anion von Benzolsulfonsäure, Naphthalin-sulfonsäure, Biphenyl-sulfonsäure, bevorzugt von Benzolsulfonsäure.

X^{⊖} ist bevorzugt Alkylsulfat, besonders bevorzugt Methylsulfat.

M¹ ist ein Äquivalent eines Alkalimetall- oder Erdalkalimetallkations, beispielsweise das Kation von Lithium, Natrium, Kalium, Rubidium, Cäsium, Magnesium, Calcium, Strontium, Barium; bevorzugt das Kation eines Alkalimetalls, besonders bevorzugt das Kation von Natrium oder Kalium.

Für den Fall, daß R¹⁰ für C₂-C₈-Alkylen-OM¹ steht, handelt es sich um das Alkoholat eines Diols mit 2 bis 8 C-Atomen, wie Glykol, 1,2-Propandiol, 1,3-Propandiol, 1,2-, 1,3- oder 1,4-Butandiol, Hexandiol oder Octandiol.

Unter die zur erfindungsgemäßen Umsetzung benutzten Salze der Formel (III) fallen Alkoxymethyleniminiumsalze der Formel 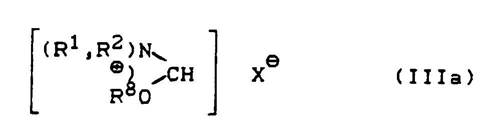
und Formamidiniumsalze der Formel 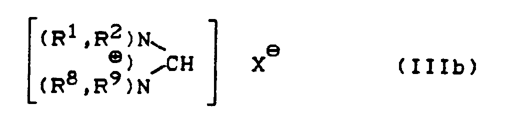
mit den oben angegebenen Substituenten.

Die erfindungsgemäße Reaktion kann beispielhaft wie folgt dargestellt werden:

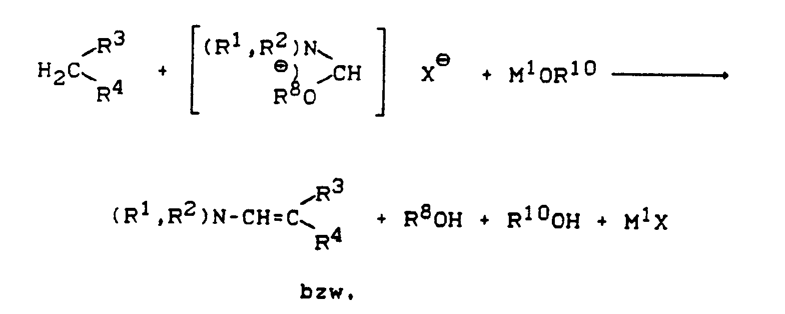

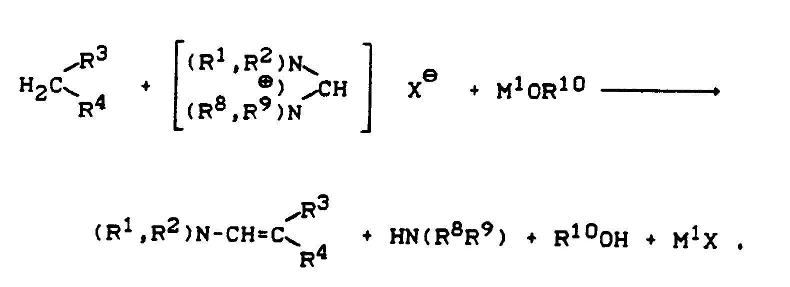

R⁸ und R⁹ haben den Bedeutungsumfang von R¹ bzw. R², sind aber unabhängig von R¹ und R². Sofern R⁸ und R⁹ verschieden von R¹ bzw. R² sind, wählt man solche Reste R⁸ und R⁹, die das sie tragende N-Atom befähigt, als HN(R⁸,R⁹) abzuspalten. Dies kann durch einfache Vorversuche festgestellt werden. In bevorzugter Weise gleichen R⁸ und R⁹ jedoch R¹ und R², so daß ein symmetrisches Formamidiniumsalz vorliegt.

In bevorzugter Weise wird eine C-H-acide Verbindung der Formel 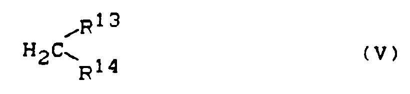
eingesetzt, in der
- R¹³ und R¹⁴: unabhängig voneinander Phenyl, NO₂, CN, COR¹⁵, COOR¹⁵ oder CO-N(R¹⁵,R¹⁶) bedeuten, wobei R¹⁵ und R¹⁶ unabhängig voneinander für Wasserstoff, geradkettiges oder verzweigtes C₁-C₈-Alkyl, Cyclopropyl, Cyclopentyl, Cyclohexyl, Phenyl oder Benzyl stehen und wobei R¹⁵ und R¹⁶ gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann.

In besonders bevorzugter Weise wird eine C-H-acide Verbindung der Formel 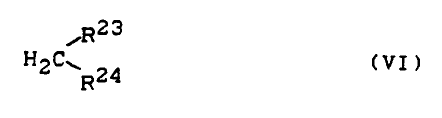
eingesetzt, in der
- R²³ und R²⁴: unabhängig voneinander Phenyl, NO₂, CN, COR²⁵, COOR²⁵ oder CO-N(R²⁵,R²⁶) bedeuten, wobei R²⁵ und R²⁶ unabhängig voneinander Wasserstoff oder geradkettiges oder verzweigtes C₁-C₄-Alkyl bedeuten und wobei weiterhin R²⁵ und R²⁶ gemeinsam mit dem N-Atom, das sie substituieren, Morpholino, Pyrrolidino oder Piperidino bedeuten können.

In weiterhin bevorzugter Weise werden die C-H-aciden Verbindungen mit einem Salz der Formel 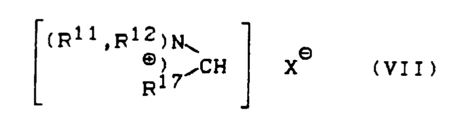
umgesetzt, in der
- R¹¹ und R¹²: unabhängig voneinander geradkettiges oder verzweigtes C₁-C₈-Alkyl, Cyclopropyl, Cyclopentyl, Cyclohexyl, Phenyl oder Benzyl bedeuten und R¹¹ und R¹² weiterhin gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann,
- R¹⁷: für -OR¹¹ oder -N(R¹¹,R¹²) steht und
- X^{⊖}: das C₁-C₈-Alkylsulfatanion, das C₆-C₁₂-Arylsulfonatanion, das Tetrafluoroboranatanion oder das Hexachloroantimonatanion bedeutet.

Weiterhin werden die C-H-aciden Verbindungen in besonders bevorzugter Weise mit einem Salz der Formel 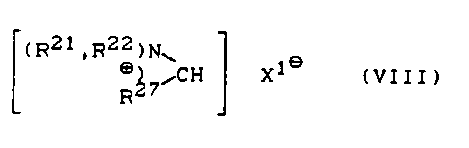
umgesetzt, in der
- R²¹ und R²²: unabhängig voneinander geradkettiges oder verzweigtes C₁-C₄-Alkyl bedeuten und R²¹ und R²² weiterhin gemeinsam mit dem N-Atom, das sie substituieren, Morpholino, Pyrrolidino oder Piperidino bedeuten können,
- R²⁷: für -OR²¹ oder -N(R²¹,R²²) steht und
- X^{1⊖}: das C₁-C₈-Alkylsulfatanion bedeutet.

In weiterhin bevorzugter Weise wird die Umsetzung in Gegenwart eines Alkoholats der Formel

M²OR²⁰ (IX)

durchgeführt, in der
- R²⁰: für geradkettiges oder verzweigtes C₁-C₅-Alkyl, C₂-C₅-Alkoxyalkyl oder C₂-C₄-Alkylen-OM² steht und
- M²: Na^{⊕} oder K^{⊕} bedeutet.

In weiterhin besonders bevorzugter Weise wird die Umsetzung in Gegenwart eines Alkoholats der Formel

M²OR³⁰ (X)

durchgeführt, in der
- R³⁰: für geradkettiges oder verzweigtes C₁-C₅-Alkyl steht und
- M²: die obige Bedeutung hat.

In ganz besonders bevorzugter Weise bedeuten die Substituenten R¹, R², R⁸, R⁹ und R¹⁰ den Methylrest. In weiterhin ganz besonders bevorzugter Weise bedeutet X^{1⊖} das C₁-C₄-Sulfatanion, bevorzugt das Methylsulfatanion.

Die C-H-acide Verbindung, das Zalz und das Alkoholat (III) werden im allgemeinen im molaren Verhältnis 1:1:1 bis 1:2,5:2 eingesetzt. In bevorzugter Weise wird das Verhältnis 1:1,1:1,05 bis 1:1,7:1,2 gewählt. Bei Malonestern kann es nötig sein, eine Salzmenge im oberen Teil des genannten Bereiches einzusetzen; bei Umsetzungen mit Malonestern fällt das aminomethylenierte Reaktionsprodukt gemeinsam mit dem nicht umgesetzten Salz als zweiphasiges System an, wodurch eine Abtrennung und Rückführung des Salzes vereinfacht wird.

Es ist ein Kennzeichen des erfindungsgemäßen Verfahrens, daß das Gemisch aus dem Salz (Alkoxymethyleniminiumsalz bzw. Tetraalkylformamidiniumsalz) und der C-H-aciden Verbindung mit einer Lösung oder Suspension des Alkoholats in einer einzigen Verfahrensstufe umgesetzt wird. Hierbei wird trotz der Gefahr unübersichtlicher Nebenreaktionen der Vorteil einer beträchtlichen Steigerung der Ausbeute erzielt.

Wenn das eingesetzte Salz und die C-H-acide Verbindung nicht mischbar sind, kann man ein Lösungsmittel verwenden und die daraus resultierende Lösung oder eine durch Rühren gleichmäßig gehaltene Emulsion einsetzen. Es ist weiterhin möglich, beide Komponenten (Salz und C-H-acide Verbindung) simultan zum vorgelegten Alkoholat zuzudosieren oder Salz, C-H-acide Verbindung und Alkoholat simultan in das Reaktionsgefäß einzudosieren.

Als Lösungsmittel kommen Kohlenwasserstoffe, wie Toluol, Xylol, Cyclohexan oder Petrolether, Alkohole, Carbonylverbindungen oder Ether zum Einsatz. Solche Lösungsmittel können auch als Gemisch eingesetzt werden. Bei der Verwendung eines unpolaren Mediums (z.B. Toluol) liegt das Alkoholat im allgemeinen suspendiert darin vor; zu dieser Suspension werden in der oben geschilderten Weise die anderen Reaktionskomponenten zudosiert.

In einem solchen Falle wird das Alkoholat während der einsetzenden Reaktion verbraucht. Das entstehende Salz fällt aus und kann von dem im Reaktionsmedium gelösten Reaktionsprodukt auf einfache Weise abgetrennt werden. Arbeitet man in einem alkoholischen Medium, liegt das Alkoholat im allgemeinen in gelöster Form vor. Allerdings sind in niederen Alkoholen auch die entstehenden Salze mindestens teilweise löslich. Eine nahezu vollständige Abtrennung von entstehenden Salzen und entstehendem Reaktionsprodukt wird durch ein Gemisch eines unpolaren Lösungsmittels und der alkoholischen Alkoholatlösung erreicht.

Enthält die verwendete C-H-acide Verbindung eine Estergruppe, ist es vorteilhaft, ein Alkoholat zu nehmen, dessen zugrunde liegender Alkohol mit dem Esteralkohol übereinstimmt; auf diese Weise werden unerwünschte Umesterungen vermieden.

Enthält die C-H-acide Verbindung beispielsweise einen Nitrilsubstituenten, wird dieser unter den milden Reaktionsbedingungen (beispielsweise 0,5 bis 2 h und 20 bis 70°C) nicht angegriffen. Selbst höhere Temperaturen sind kurzzeitig, beispielsweise zum Andestillieren, möglich.

Setzt man die Methylsulfatsalze der Alkoxymethyleniminiumionen bzw. Formamidiniumionen ein, so wird auch bei höherer Temperatur das eingesetzte Alkoholat durch das Anion überraschenderweise nicht methyliert, wodurch Ausbeuteverminderungen vermieden werden.

Die Ausbeuten im erfindungsgemäßen Verfahren liegen mit etwa 90 bis 98 % der theoretischen Ausbeute recht hoch; hierdurch ist das Reaktionsprodukt sehr rein und kann für weitere Umsetzungen im allgemeinen direkt verwendet werden. Mit den Formamidiniumsalzen lassen sich je nach C-H-acider Verbindung um 1 bis 20 % höhere Ausbeuten erzielen als mit den Alkoxymethyleniminiumsalzen. Da sich die ersteren aus den letzteren durch Umsetzung mit einem sekundären Amin in nahezu quantitativer Ausbeute herstellen lassen, kann je nach Aminomethylenierungsausbeute das kostengünstigere Verfahren gewählt werden.

### Beispiel 1

Die Emulsion von 66,0 g Malonsäuredimethylester und 116,6 g Tetramethylformamidinium-methylsulfat wurden in 300 ml trockenem Toluol vorgelegt. Innerhalb von 10 Min. tropfte man unter Feuchtigkeitsausschluß 104 ml 30 %ige Natriummethylatlösung zu und rührte 1 h bei Raumtemperatur. Anschließend saugte man vom gebildeten Natriummethylsulfat (67,4 g) ab, wusch zweimal mit je 30 ml Toluol nach und engte am Rotationsverdampfer ein. Es wurden 104,5 g 84,1 %iger Dimethylaminomethylenmalonester erhalten, was 94,0 % der theoretischen Ausbeute entspricht. Das Produkt konnte roh ohne Nachteile gegenüber destillierter Ware für eine weitere Umsetzung verwendet werden.

### Beispiel 2

Zu einer Lösung von 55,3 g Tetramethylformamidiniummethylsulfat und 43,2 g Malonsäurediisobutylester tropfte man 68,5 g einer etwa 30 %igen Natriumisobutylatlösung, die zusätzlich mit 200 ml Isobutanol verdünnt war. Nach 90 Min. bei 70°C wurde vom Salz abgesaugt, zweimal mit je 20 ml Isobutanol nachgewaschen und am Rotationsverdampfer eingeengt. Die untere Phase, die hauptsächlich aus dem Formamidiniumsalz bestand, wurde mit Hilfe einer Pipette entfernt. Der Rückstand (53,8 g) bestand zu 98,8 % aus Dimethylaminomethylenmalonester, was 98,1 % der theoretischen Ausbeute entspricht.

### Beispiel 3

Analog Beispiel 2 wurde mit 54 g Dimethylformamid-Dimethylsulfat-Addukt aminomethyleniert. Man tropfte bei 0°C zu und erwärmte dann 3 h auf 50°C. Es wurden 53,5 g 91,6 %iges Produkt erhalten, entsprechend einer Ausbeute von 90,4 % der theoretischen Ausbeute.

### Beispiel 4

Zu einer Suspension von 7,5 g Natriumethylat in 100 ml trockenem Toluol tropfte man ein Gemisch aus 25,4 g Tetramethylformamidinium-methylsulfat und 11,3 g Cyanessigsäureethylester. Nach 1 h bei 40°C saugte man bei 50°C vom Natriummethylsulfat ab, wusch zweimal mit je 15 ml Toluol und engte am Rotationsverdampfer ein. Es wurden 17,2 g 94,3 %iges hellgelbes Produkt erhalten, entsprechend 98,5 % der theoretischen Ausbeute. Nach Umkristallisation aus wäßrigem Isopropanol konnten weiße Kristalle vom Schmelzpunkt 80°C erhalten werden.

### Beispiel 5

Entsprechend Beispiel 4 wurden 9,9 g Cyanessigsäuremethylester mit 26,5 g Formamidiniumsalz und 5,4 g Natriummethylat in 180 ml Toluol umgesetzt. Man erhielt 15,0 g 92,7 %iges Produkt, entsprechend 93,2 % der theoretischen Ausbeute. Das abgesaugte Natriummethylsulfat enthielt weitere 2 % der theoretischen Ausbeute.

### Beispiel 6 (zum Vergleich)

Zu 0,5 Mol einer 3-molaren Natriummethylatlösung in Ethanol tropfte man bei 0°C 0,5 Mol DMF-Dimethylsulfat-Addukt, Nach 2 h Rühren bei 20°C destillierte man die Hauptmenge ab und destillierte dann unter Zutropfen von Ethanol zum Salzbrei weiter. Nachdem 300 ml Ethanol rundgeführt worden waren, fraktionierte man das Destillat mit Hilfe einer Füllkörperkolonne. Es wurden 47 g DMF-Acetal erhalten, entsprechend 63,9 % Ausbeute.

In einem Rührkolben mit Kolonne wurden 147 g DMF-Diethylacetal zusammen mit 149 g Malonsäurediethylester 4 h auf 140 bis 150°C erhitzt. Dabei destillierten 82 g Ethanol über. Das Reaktionsgemisch wurde anschließend im Hochvakuum fraktioniert und 164 g, entsprechend 82 % der theoretischen Ausbeute an Dimethylaminomethylenmalonester, erhalten.

## Patentansprüche

1. Verfahren zur Herstellung von Aminomethylenverbindungen der Formel 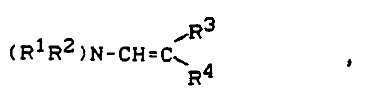 in der
R¹ und R² unabhängig voneinander geradkettiges oder verzweigtes C₁-C₈-Alkyl, C₂-C₈-Alkenyl, C₂-C₈-Alkoxyalkyl,C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R¹ und R² gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann, und
R³ und R⁴ unabhängig voneinander C₆-C₁₂-Aryl, -NO₂, -CN, -NC, COR⁵, CSR⁵, CO-OR⁵, CO-SR⁵ oder CO-N(R⁵,R⁶) bedeuten, worin R⁵ und R⁶ den für R¹ und R² genannten Bedeutungsumfang annehmen, jedoch unabhängig von R¹ und R² sind und zusätzlich Wasserstoff bedeuten können,
dadurch gekennzeichnet, daß man C-H-acide Verbindungen der Formel 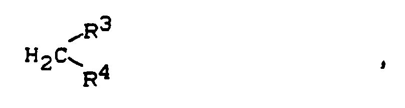 in der
R³ und R⁴ die genannte Bedeutung haben,
mit Salzen der Formel 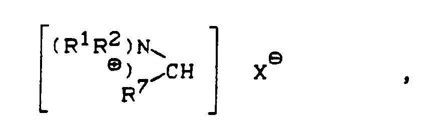 in der
R¹ und R² die genannte Bedeutung haben,
R⁷ für -OR⁸ oder -N(R⁸,R⁹) stehen, worin R⁸ und R⁹ unabhängig voneinander und unabhängig von R¹ und R² den für R¹ und R² genannten Bedeutungsumfang haben und
X^{⊖} das C₁-C₈-Alkylsulfatanion, das C₆-C₁₂-Arylsulfonatanion, das Tetrafluoroboranatanion oder das Hexachloroantimonatanion bedeutet,
in Gegenwart von Alkoholaten der Formel
M¹OR¹⁰ ,
in der
R¹⁰ geradkettiges oder verzweigtes C₁-C₈-Alkyl, C₂-C₈-Alkenyl, C₂-C₈-Alkoxyalkyl, C₃-C₈-Alkoxyalkenyl C₃-C₈-Cycloalkyl, C₂-C₈-Alkylen-OM¹ oder C₇-C₁₀-Aralkyl bedeutet und
M¹ ein Äquivalent eines Alkalimetall- oder Erdalkalimetallkations ist,
in einer einstufigen Reaktion bei einer Temperatur von 10 bis 70°C, bevorzugt 20 bis 60°C, umsetzt.

2. Verfahren nach Anspruch 1, dadurch gekennzeichnet, daß eine C-H-acide Verbindung der Formel 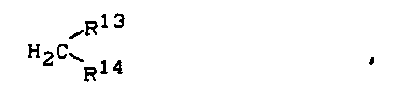 eingesetzt, in der
R¹³ und R¹⁴ unabhängig voneinander Phenyl, NO₂, CN, COR¹⁵, COOR¹⁵ oder CO-N(R¹⁵,R¹⁶) bedeuten, wobei R¹⁵ und R¹⁶ unabhängig voneinander für Wasserstoff, geradkettiges oder verzweigtes C₁-C₈-Alkyl, Cyclopropyl, Cyclopentyl, Cyclohexyl, Phenyl oder Benzyl stehen und wobei R¹⁵ und R¹⁶ gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann.

3. Verfahren nach Anspruch 2, dadurch gekennzeichnet, daß eine C-H-acide Verbindung der Formel 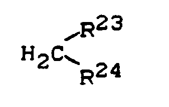 eingesetzt wird, in der
R²³ und R²⁴ unabhängig voneinander Phenyl, NO₂, CN, COR²⁵, COOR²⁵ oder CO-N(R²⁵,R²⁶) bedeuten, wobei R²⁵ und R²⁶ unabhängig voneinander Wasserstoff, geradkettiges oder verzweigtes C₁-C₄-Alkyl bedeuten und wobei weiterhin R²⁵ und R²⁶ gemeinsam mit dem N-Atom, das sie substituieren, Morpholino, Pyrrolidino oder Piperidino bedeuten können.

4. Verfahren nach Anspruch 1, dadurch gekennzeichnet, daß die C-H-aciden Verbindungen mit Salzen der Formel 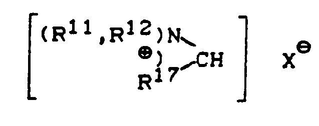 umgesetzt werden, in der
R¹¹ und R¹² unabhängig voneinander geradkettiges oder verzweigtes C₁-C₈-Alkyl, Cyclopropyl, Cyclopentyl, Cyclohexyl, Phenyl oder Benzyl bedeuten und R¹¹ und R¹² weiterhin gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann,
R¹⁷ für -OR¹¹ oder -N(R¹¹,R¹²) steht und
X^{⊖} das C₁-C₈-Alkylsulfatanion, das C₆-C₁₂-Arylsulfonatanion, das Tetrafluoroboranatanion oder das Hexachloroantimonatanion bedeutet.

5. Verfahren nach Anspruch 4, dadurch gekennzeichnet, daß die C-H-aciden Verbindungen mit Salzen der Formel 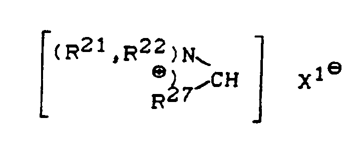 umgesetzt werden, in der
R²¹ und R²² unabhängig voneinander geradkettiges oder verzweigtes C₁-C₄-Alkyl bedeuten und R²¹ und R²² weiterhin gemeinsam mit dem N-Atom, das sie substituieren, Morpholino, Pyrrolidino oder Piperidino bedeuten können,
R²⁷ für -OR²¹ oder -N(R²¹,R²²) steht und
X^{1⊖} das C₁-C₈-Alkylsulfatanion bedeutet.

6. Verfahren nach Anspruch 1, dadurch gekennzeichnet, daß in Gegenwart von Alkoholaten der Formel
M²OR²⁰
gearbeitet wird, in der
R²⁰ für geradkettiges oder verzweigtes C₁-C₅-Alkyl, C₂-C₅-Alkoxyalkyl oder C₂-C₄-Alkylen -OM² steht und
M² Na^{⊕} oder K^{⊕} bedeutet.

7. Verfahren nach Anspruch 6, dadurch gekennzeichnet, daß in Gegenwart von Alkoholaten der Formel
M²OR³⁰
gearbeitet wird, in der
R³⁰ für geradkettiges oder verzweigtes C₁-C₅-Alkyl steht und
M² Na^{⊕} oder K^{⊕} bedeutet.

8. Verfahren nach Anspruch 1, dadurch gekennzeichnet, daß die C-H-acide Verbindung, das Salz und das Alkoholat in einem molaren Verhältnis von 1:1:1 bis 1:2,5:2, bevorzugt 1:1,1:1,05 bis 1:1,7:1,2, eingesetzt werden.

9. Verfahren nach Anspruch 1, dadurch gekennzeichnet, daß als Salze die Formamidiniumsalze eingesetzt werden.

## Claims

1. Process for the preparation of aminomethylene compounds of the formula 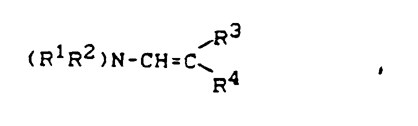 in which
R¹ and R² independently of one another represent straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₆-C₁₂-aryl, C₇-C₁₀-aralkyl or a 5- to 8-membered saturated or unsaturated heterocyclic ring, heteroatoms 1 or 2 of which are from the group comprising N, O and S, it furthermore being possible for R¹ and R², together with the N atom which they substitute, to form a 5- to 8-membered saturated or unsaturated N-heterocyclic ring which can contain a further heteroatom from the group comprising N, O and S, and
R³ and R⁴ independently of one another denote C₆-C₁₂-aryl, -NO₂, -CN, -NC, COR⁵, CSR⁵, CO-OR⁵, CO-SR⁵ or CO-N(R⁵,R⁶), in which R⁵ and R⁶ assume the scope of meaning given for R¹ and R², but are independent of R¹ and R² and can additionally denote hydrogen,
characterized in that C-H acid compounds of the formula 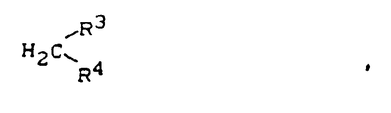 in which
R³ and R⁴ have the meaning mentioned,
are reacted with salts of the formula 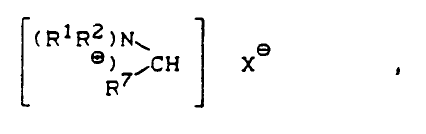 in which
R¹ and R² have the meaning mentioned,
R⁷ represents -OR⁸ or -N(R⁸,R⁹), in which R⁸ and R⁹ independently of one another and independently of R¹ and R² have the scope of meaning mentioned for R¹ and R² and
X^{⊖} denotes the C₁-C₈-alkylsulphate anion, the C₆-C₁₂-arylsulphonate anion, the tetrafluoroborate anion or the hexachloroantimonate anion,
in the presence of alkoxides of the formula
M¹OR¹⁰
in which
R¹⁰ denotes straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₂-C₈-alkylene-OM¹ or C₇-C₁₀-aralkyl and
M¹ is an equivalent of an alkali metal cation or an alkaline earth metal cation,
in a one-step reaction at a temperature of 10 to 70°C, preferably 20 to 60°C.

2. Process according to Claim 1, characterized in that a C-H acid compound of the formula 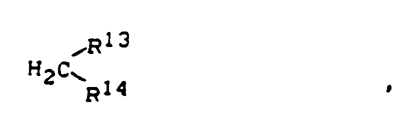 is employed in which
R¹³ and R¹⁴ independently of one another denote phenyl, NO₂, CN, COR¹⁵, COOR¹⁵ or CO-N(R¹⁵,R¹⁶), where R¹⁵ and R¹⁶ independently of one another represent hydrogen, straight-chain or branched C₁-C₈-alkyl, cyclopropyl, cyclopentyl, cyclohexyl, phenyl or benzyl and R¹⁵ and R¹⁶, together with the N atom which they substitute, can form a 5- to 8-membered saturated or unsaturated N-heterocyclic ring which can contain a further heteroatom from the group comprising N, O and S.

3. Process according to Claim 2, characterized in that a C-H acid compound of the formula 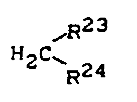 is employed in which
R²³ and R²⁴ independently of one another denote phenyl, NO₂, CN, COR²⁵, COOR²⁵ or CO-N(R²⁵,R²⁶), where R²⁵ and R²⁶ independently of one another denote hydrogen or straight-chain or branched C₁-C₄-alkyl and furthermore where R²⁵ and R²⁶, together with the N atom which they substitute, can denote morpholino, pyrrolidino or piperidino.

4. Process according to Claim 1, characterized in that the C-H acid compounds are reacted with salts of the formula 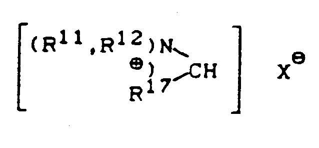 in which
R¹¹ and R¹² independently of one another denote straight-chain or branched C₁-C₈-alkyl, cyclopropyl, cyclopentyl, cyclohexyl, phenyl or benzyl and R¹¹ and R¹² furthermore, together with the N atom which they substitute, can form a 5- to 8-membered saturated or unsaturated N-heterocyclic ring which can contain a further heteroatom from the group comprising N, O and S,
R¹⁷ represents -OR¹¹ or -N(R¹¹,R¹²) and
X^{⊖} denotes the C₁-C₈-alkylsulphate anion, the C₆-C₁₂-arylsulphonate anion, the tetrafluoroborate anion or the hexachloroantimonate anion.

5. Process according to Claim 4, characterized in that the C-H acid compounds are reacted with salts of the formula 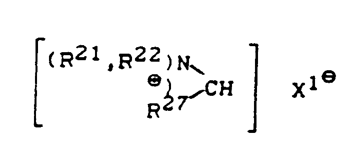 in which
R²¹ and R²² independently of one another denote straight-chain or branched C₁-C₄-alkyl and R²¹ and R²² furthermore, together with the N atom which they substitute, can denote morpholino, pyrrolidino or piperidino,
R²⁷ represents -OR²¹ or -N(R²¹,R²²) and
X^{'⊖} denotes the C₁-C₈-alkylsulphate anion.

6. Process according to Claim 1, characterized in that it is carried out in the presence of alkoxides of the formula
M²OR²⁰
in which
R²⁰ represents straight-chain or branched C₁-C₅-alkyl, C₂-C₅-alkoxyalkyl or C₂-C₄-alkylene-OM² and
M² denotes Na^{⊕} or K^{⊕}.

7. Process according to Claim 6, characterized in that it is carried out in the presence of alkoxides of the formula
M²OR³⁰
in which
R³⁰ represents straight-chain or branched C₁-C₅-alkyl and
M² denotes Na^{⊕} or K^{⊕}.

8. Process according to Claim 1, characterized in that the C-H acid compound, the salt and the alkoxide are employed in a molar ratio of 1:1:1 to 1:2.5:2, preferably 1:1.1:1.05 to 1:1.7:1.2.

9. Process according to Claim 1, characterized in that the formamidinium salts are employed as salts.

## Revendications

1. Procédé de production de composés aminométhyléniques de formule 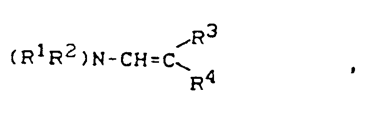 dans laquelle
R¹ et R² représentent, indépendamment l'un de l'autre, un groupe alkyle en C₁ à C₈ linéaire ou ramifié, alcényle en C₂ à C₈, alkoxyalkyle en C₂ à C₈, alkoxyalcényle en C₃ à C₈, cycloalkyle en C₃ à C₈, aryle en C₆ à C₁₂, aralkyle en C₇ à C₁₀ ou un noyau hétérocyclique pentagonal à octogonal saturé ou non saturé, dont les hétéro-atomes représentent 1 ou 2 atomes du groupe N, O et S, R¹ et R² pouvant en outre former conjointement avec l'atome d'azote qu'ils substituent un noyau hétérocyclique azoté saturé ou non saturé pentagonal à octogonal qui peut comporter un autre hétéro-atome du groupe N, O et S, et
R³ et R⁴ représentent, indépendamment l'un de l'autre, un groupe aryle en C₆ à C₁₂, -NO₂, -CN, -NC, COR⁵, CSR⁵, CO-OR⁵, CO-SR⁵ ou CO-N(R⁵,R⁶) où R⁵ et R⁶ rentrent dans le cadre de la définition mentionnée pour R¹ et R², en étant toutefois indépendants de R¹ et R² et pouvant en outre représenter l'hydrogène,
caractérisé en ce qu'on fait réagir à une température de 10 à 70°C, de préférence de 20 à 60°C, dans une réaction à une seule étape, des composés C-H-acides de formule 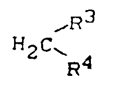 dans laquelle
R³ et R⁴ ont la définition mentionnée,
avec des sels de formule 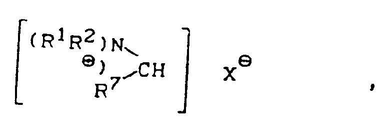 dans laquelle
R¹ et R² ont la définition mentionnée,
R⁷ représente -OR⁸ ou -N(R⁸,R⁹) où R⁸ et R⁹ ont, indépendamment l'un de l'autre et indépendamment de R¹ et R², la définition mentionnée pour R¹ et R² et
X^{⊖} est l'anion (alkyle en C₁ à C₈)-sulfate, l'anion (aryle en C₆ à C₁₂)-sulfonate, l'anion tétrafluoroboranate ou l'anion hexachlorantimoniate,
en présence d'alcoolates de formule
M¹OR¹⁰
dans laquelle
R¹⁰ est un groupe alkyle en C₁ à C₈ linéaire ou ramifié, alcényle en C₂ à C₈, alkoxyalkyle en C₂ à C₈, alkoxyalcényle en C₃ à C₈, cycloalkyle en C₃ à C₈, (alkylène en C₂ à C₈)-OM¹ ou aralkyle en C₇ à C₁₀ et
M¹ est un équivalent d'un cation de métal alcalin ou de métal alcalino-terreux.

2. Procédé suivant la revendication 1, caractérisé en ce qu'on utilise un composé C-H-acide de formule 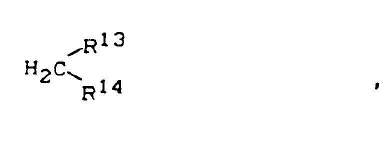 dans laquelle
R¹³ et R¹⁴ représentent, indépendamment l'un de l'autre, un groupe phényle, NO₂, CN, COR¹⁵, COOR¹⁵ ou CO-N(R¹⁵,R⁶), où R¹⁵ et R¹⁶ représentent, indépendamment l'un de l'autre, l'hydrogène, un groupe alkyle en C₁ à C₈ linéaire ou ramifié, cyclopropyle, cyclopentyle, cyclohexyle, phényle ou benzyle et R¹⁵ et R¹⁶ peuvent former conjointement avec l'atome d'azote qu'ils substituent un noyau hétérocyclique azoté saturé ou non saturé pentagonal à octogonal qui peut comporter un autre hétéro-atome du groupe N, O et S.

3. Procédé suivant la revendication 2, caractérisé en ce qu'on utilise un composé C-H-acide de formule 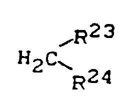 dans laquelle
R²³ et R²⁴ représentent, indépendamment l'un de l'autre, un groupe phényle, NO₂, CN, COR²⁵, COOR²⁵ ou CO-N(R²⁵,R²⁶), où R²⁵ et R²⁶ représentent, indépendamment l'un de l'autre, l'hydrogène, un groupe alkyle en C₁ à C₄ linéaire ou ramifié et, en outre, R²⁵ et R²⁶ peuvent former conjointement avec l'atome d'azote qu'ils substituent un groupe morpholino, pyrrolidino ou pipéridino.

4. Procédé suivant la revendication 1, caractérisé en ce que les composés C-H-acides sont amenés à réagir avec des sels de formule 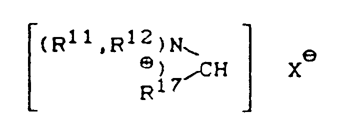 dans laquelle
R¹¹ et R¹² représentent, indépendamment l'un de l'autre, un groupe alkyle en C₁ à C₈ linéaire ou ramifié, cyclopropyle, cyclopentyle, cyclohexyle, phényle ou benzyle et R¹¹ et R¹² peuvent en outre former conjointement avec l'atome d'azote qu'ils substituent un noyau hétérocyclique azoté saturé ou non saturé pentagonal à octogonal qui peut comporter un autre hétéro-atome du groupe N, O et S,
R¹⁷ est un groupe -OR¹¹ ou -N(R¹¹,R¹²) et
X^{⊖} désigne l'anion (alkyle en C₁ à C₈)-sulfate, l'anion (aryle en C₆ à C₁₂)-sulfonate, l'anion tétrafluoroboranate ou l'anion hexachlorantimoniate.

5. Procédé suivant la revendication 4, caractérisé en ce qu'on fait réagir les composés C-H-acides avec des sels de formule 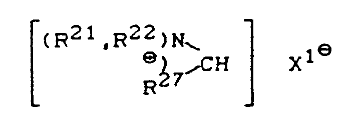 dans laquelle
R²¹ et R²² représentent, indépendamment l'un de l'autre, un groupe alkyle en C₁ à C₄ linéaire ou ramifié et R²¹ et R²² peuvent en outre former conjointement avec l'atome d'azote qu'ils substituent un noyau morpholino, pyrrolidino ou pipéridino,
R²⁷ représente un groupe -OR²¹ ou -N(R²¹,R²²) et
X^{1⊖} désigne l'anion (alkyle en C₁ à C₈)-sulfate.

6. Procédé suivant la revendication 1, caractérisé en ce qu'on opère en présence d'alcoolates de formule
M²OR²⁰
dans laquelle
R²⁰ est un groupe alkyle en C₁ à C₅ linéaire ou ramifié, un groupe alkoxyalkyle en C₂ à C₅ ou un groupe (alkylène en C₂ à C₄)-OM² et
M² représente Na^{⊕} ou K^{⊕}.

7. Procédé suivant la revendication 6, caractérisé en ce qu'on opère en présence d'alcoolates de formule
M²OR³⁰
dans laquelle
R³⁰ est un groupe alkyle en C₁ à C₅ linéaire ou ramifié et
M² représente Na^{⊕} ou K^{⊕}.

8. Procédé suivant la revendication 1, caractérisé en ce qu'on utilise le composé C-H-acide, le sel et l'alcoolate dans une proportion molaire de 1:1:1 à 1:2,5:2, de préférence de 1:1,1:1,05 à 1:1,7:1,2.

9. Procédé suivant la revendication 1, caractérisé en ce qu'on utilise comme sels des sels de formamidinium.
